# EUROPEAN PATENT APPLICATION

(11) **EP 2 316 497 A2**
(43) Date of publication of application: **04.05.2011**
(21) Application number: 10181752.6
(22) Date of filing: 06.01.2006
(51) Int. Cl.: A61L 27/22, A61L 27/12

(54) **Supplemented matrices for the repair of bone fractures**

(30) Priority: 06.01.2005 US 641715 P; 10.01.2005 US 642644 P
(62) Divisional of application: 06707674.5
(71) Applicant: Kuros Biosurgery AG, 8005 Zürich (CH)
(72) Inventor: Schense, Jason, 8008, Zürich (CH); Watson, John, 8126, Zumikon (CH); Arrighi, Isabelle, 8006, Zurich (CH)
(74) Representative: Wilming, Martin

(57) **Abstract**

Supplemented matrices comprising a PTH releasably incorporated therein, optionally containing a granular material, are described herein. The PTH is incorporated either through covalent linkage to the matrix or through non-covalent interaction with the matrix and/or the granules. These supplemented matrices decrease the time of healing compared to autograft and or trigger healing of bone fractures which otherwise would not heal. The matrices are biocompatible and biodegradable and can be formed *in vitro* or *in vivo*, at the time of implantation. The PTH may be a part of a fusion peptide. PTH can be incorporated into the matrices with full retention of its bioactivity. PTH can be releasably incorporated, using techniques that provide control over how and when and to what degree the PTH is released using the matrix as a controlled release vehicle to heal bone fractures.

## Description

### Field of the Invention

The present application relates to supplemented matrices and uses thereof for the repair and healing of bone fractures.

### Background of the Invention

In both Europe and the United States an estimated 5 to 6 million people sustain bone fractures each year due to trauma, sports- or activity-related injuries or osteoporosis Most of these injuries may be treated with manual reduction and external fixation (e.g. a cast). However approximately 20 to 25% of fractures require hospitalization, usually with open surgical procedures.

A fracture is defined as a discontinuity in the cortical bone structure, usually resulting from excess mechanical force. A complete fracture is defined by a discontinuity, or break, that occurs across the entire bone structure, creating two or more distinct bone segments. The bone fragments do not have to separate for the injury to be classified as a fracture. In some cases the periosteum holds the bone fragments in their original anatomical position. In other cases, the fracture causes the limb to bend, tearing the periosteum or even the surrounding skin and muscle tissue. In still other cases, as is often the case with high-velocity missile injuries or serious accidents, large portions of bone are fragmented or even removed from its original location. The primary goals of fracture treatments are sound union and the restoration of bone function without an outcome of deformity. Obtaining these goals quickly is an increasingly important concern due to disability issues and cost-containment. In a significant part of the patient population both goals, i.e. sound unions and fast restoration of bone function, are at risk due to the patient's age and/or general health condition, and/or the type and/or location of fracture. In particular, in case of osteoporotic patients, the risk of non-unions and increased healing times is high. As a disease of the skeleton, osteoporosis is characterised by low bone mass and the structural deterioration of bone tissue leading to increased bone fragility, increased healing times and the occurrence of non-union.

Bone grafts and bone graft substitutes are widely used in many orthopaedic procedures to treat problems associated with bone loss, delayed union and non-union fractures or as an implant fixation material. In case of severe and complicated fractures, bone graft and bone graft substitutes are used to strengthen bone before the fracture is then stabilized with hardware. Bone grafting materials may be autogenic, allogenic, xenogenic, demineralised bone matrix (DBM), of synthetic origin and mixtures thereof. Bone grafting materials can be classified into materials with osteoconductive, osteoinductive and ostoeogenic properties. Osteoconductive materials do not create bone; rather they simulate the migration of nearby living bone cells into the material. Osteoinductive materials stimulate the patient's own system to generate bone tissue. Osteogenic materials directly create bone tissue. Osteogenic materials are confined to osteoblasts and mesenchymal stem cells that generate bone tissue. Some materials exhibit more than one of the described properties.

Autograft refers to any tissue or bone that is harvested from one part of the patient's body to be used at the injury site. Bone autografts are usually harvested from the iliac crest. In spite their advantage of being biocompatible, safe, of a vascularized composition and exhibiting osteoinductive properties, the disadvantages are major. Autografts require a second operation which may lead to postoperative complications which include blood-loss, infections, and pain. Autografts are costly due to longer hospital stays and operation time. The supply of autografts per patient is limited, and the post operative pain after harvesting of autogenic material is often higher than the bone fracture itself. In spite of the disadvantages, autograft is considered the "gold standard" in terms of bone grafting materials. Allografts are bone tissue taken from various locations in a human cadaver body, which can be machined with different structures and into different shapes. Allografts are minimally osteoinductive, there is only limited supply, and they pose on the patient the risk of infections due to host pathogens. Synthetic bone graft materials are developed as an "off-the-shelf" alternative to autogenic bone grafting material. Synthetic bone graft substitute include ceramic materials and self-setting polymers that mimic properties of human bone, like hydroxyapatite and polymethylmethacrylate, collagen, tricalciumphosphate, calciumsulfates and -phosphates. These materials show poor handling properties and a lack of osteoinductive properties.

In recent years efforts have been made to develop bone graft substitutes which show osteoinductive properties as a true "off-the-shelf alternative" to autografts. DBM is one example of an osteoinductive bone graft substitute. However DBM as an allogenic material faces the same drawbacks like allogenic bone. Other examples are Stryker Corp.'s OP-1^{®}, which delivers recombinantly produced bone morphogenic protein 7 (BMP 7) from a collagen matrix or Medtronic So-famor Danek's INFLTSE^{®} bone graft substitute material using recombinantly produced BMP2 from collagen sponges. Apart from the expensive and lengthy production process of the BMPs, the proteins in both products are delivered from a collagen matrix in high concentrations. Collagen matrices from bovine origin carry all the risks of xenogenic materials, show poor handling properties in the surgical procedure, e.g. are they not moldable to closely fit to the shape of the injury site, and the high concentration of BMPs delivered to the body can lead in some of the patients to calcification of organs or to bone formation in other parts of the body.

Other matrices for the local treatment of bone defects, include using human parathyroid hormone (PTH) or its derivatives which are delivered to the body as in an injectable *in situ* forming fibrin sealant composition. Parathyroid hormone is an 84 amino acid peptide that is made and secreted by the parathyroid gland The full length hormone serves in regulation of systemic calcium levels and bone turnover. Parathyroid hormone is a potent direct anabolic factor for precursor osteoblasts and an indirect anabolic factor for osteoclasts. By regulating the balance of osteoblasts and osteoclasts, parathyroid hormone has a direct effect on the resulting bone turnover and subsequent liberation of calcium into the body. Many animal studies have pointed to the potential anabolic effects of parathyroid hormone on bone mineral density (BMD). The parathyroid hormone acts on cells by binding to a cell surface receptor. This receptor is known to be found on osteoblasts, the cells that are responsible for forming new bone.

The N-terminal 34 amino acid domain of the human parathyroid hormone has been reported to be biologically equivalent to the full length hormone. Parathyroid hormone 1-34 and its mode of action have been first reported in U.S. Patent No. 4,086,196. Parathyroid hormone ₁₋₃₄ is known to be a fully active truncated version of parathyroid hormone which does not have disulfide bonds or significant tertiary structure. It contains a moderate secondary structure, including several alpha helices. Many clinical studies have been carried out using systemically administered parathyroid hormone to increase the overall bone mass in patients with osteoporosis, with the majority requiring daily injections of parathyroid hormone or parathyroid hormone ₁₋₃₄ alone, or in combination with other actives, for many months. More details about PTH and parathyroid hormone 1to 34 are described in WO 03/052091, the respective content thereof being incorporated herein by reference. Other truncated versions of parathyroid hormone with biological activity include parathyroid hormone 1-25, 1-31 and 1-38.

While much work has been done studying the systemic effects of PTH, the local administration ofPTH has barely been explored. WO 03/052091 describes matrices for local administration of PTH. Specifically, WO 03/052091 describes parathyroid hormone as being covalently attached to synthetic and natural matrices, in particular fibrin or polyethyleneglycol matricess, for local administration and release at the site of need in a controlled fashion.

However, WO 03/052091 does not describe matrices which are suitable for the healing of bone fracture, in particular for severe bone fractures, like repair of fractures at risk of becoming delayed unions or non-unions.

It is therefore, an object of the present invention to provide a matrix which is suitable for the local repair of bone fractures.

### Summary of the Invention

It has been surprisingly found that a matrix containing PTH ("supplemented matrices") can be used to locally deliver PTH to the site of a bone fracture to repair and heal the fracture.

Thus, the present invention is related to a formulation comprising
(i) a peptide selected from the group consisting of PTH and a PTH fusion peptide;
(ii) a granular material comprising a calcium mineral and
(iii) a composition capable of forming a fibrin matrix under physiological conditions comprising a fibrinogen precursor component and thrombin precursor component,
wherein the PTH or PTH fusion peptide is present in a concentration range of between 0.01 to 2 mg/mL fibrin matrix or precursor components forming the matrix, with the proviso that a concentration of 0.4mg/mL fibrin matrix or precursor components forming the matrix is not included.

The present invention is also related to a supplemented matrix comprising
(i) PTH or a PTH fusion peptide;
(ii) a granular material comprising a calcium mineral and
(iii) fibrin;
wherein said PTH or PTH fusion peptide is present in a concentration range of between 0.01 to 2 mg/mL fibrin matrix, with the proviso that a concentration of 0.4mg/mL fibrin matrix is not included.

The present invention is also related to the use of said formulation and supplemented matrix for the manufacture of a medicament to be locally administered for the repair of bone fractures.

Preferably the PTH is releasably incorporated in the matrix and the supplemented matrix is applied or formed at the site of the fracture. Preferably the PTH is covalently bound to the matrix. In one embodiment the matrix is a fibrin matrix.. The parathyroid hormone can be PTH₁₋₈₄ (native), PTH₁₋₃₈, PTH₁₋₃₄, PTH₁₋₃₁, or PTH₁₋₂₅, or any modified or allelic versions of PTH exhibiting properties, i.e. bone formation, similar to the foregoing ("PTH"). Preferably the PTH is PTH₁₋₃₄. In a preferred embodiment, the PTH is a fusion peptide ("PTH fusion peptide") containing at least two domains wherein the first domain comprises PTH and the second domain comprises a covalently crosslinkable substrate domain able to crosslink to the matrix during or after its formation.

In one embodiment, a supplemented fibrin matrix is formed from a formulation comprising (i) a granular material, (ii) a composition suitable of forming a fibrin matrix containing fibrinogen and thrombin and (iii) PTH in a range of between 0.01 to 2 mg PTH/mL fibrin matrix, which is suitable for the repair and healing of bone fractures with the proviso that a concentration of 0.4mg PTH/mL fibrin matrix is not included

A kit comprising the above formulation is also provided, wherein at least one of the components suitable of forming a matrix is stored separately from the other components for forming the matrix.

The formulations and supplemented matrices are preferably used for the repair and healing of bone fractures, in particular for bone fractures with a risk of becoming delayed unions or non-unions. Preferred indications include fractures of the wrist (distal radius fractures), long bone fractures, like tibia, and hip fractures.

### Brief Description of the Drawings

FIG. 1 shows the bioactivity of PTH variants. Cells transfected with a reporter gene linked to a promoter for a PTH receptor were treated with equal amounts of either PTH₁₋₃₄, TG-pl-PTH₁₋₃₄ (described hereinafter) or the international 84 amino acid standard PTH. The inhibition of expression of the luciferase reporter gene was measured and compared to transfected cells that were not exposed to PTH in solution (control).
FIG. 2 shows the results of a PTH release assay from a fibrin matrix.
FIG. 3 shows the results of a stability test of segmental tibial defects upon treatment with the supplemented matrix, presented as the score of healing.
FIG. 4 shows the results of a stability test of segmental tibial defects upon treatment with the supplemented matrix, presented in percent of remaining non-stable joints.

### Detailed Description of the Invention

Supplemented matrices comprising a PTH releasably incorporated therein, optionally containing a granular material, are described herein. The PTH is incorporated either through covalent linkage to the matrix or through non-covalent interaction with the matrix and/or the granules. These supplemented matrices decrease the time of healing compared to autograft and or trigger healing of bone fractures which otherwise would not heal. The matrices are biocompatible and biodegradable and can be formed *in vitro* or *in vivo*, at the time of implantation. PTH can be incorporated into the matrices with full retention of its bioactivity. PTH can be releasably incorporated, using techniques that provide control over how and when and to what degree the PTH is released using the matrix as a controlled release vehicle to heal bone fractures.

### Definitions

*"Adhesion site or cell attachment site"* as generally used herein refers to a peptide sequence to which a molecule, for example, an adhesion-promoting receptor on the surface of a cell, binds. Examples of adhesion sites include, but are not limited to, the RGD sequence from fibronectin, and the YIGSR (SEQ ID NO: 1) sequence from laminin. Adhesion sites can be optionally incorporated into the matrix by including a substrate domain crosslinkable to the fibrin matrix.

*"Biological activity"* as generally used herein refers to functional events mediated by a protein of interest. In some embodiments, this includes events assayed by measuring the interactions of a polypeptide with another polypeptide. It also includes assaying the effect which the protein of interest has on cell growth, differentiation, death, migration, adhesion, interactions with other proteins, enzymatic activity, protein phosphorylation or dephosphorylation, transcription, or translation.

*"Bone fracture"* as used herein refers to a discontinuity or break across the entire bone structure creating two or more distinct bone segments.

*"Calcium mineral"* as generally used herein refers to naturally occurring homogenous substances which contain calcium ions. An example for a calcium mineral is hydroxyapatite (Ca₅[(OH)(PO₄)₃]), which is the main component of teeth and bones.

*"Cross-linking"* as generally used herein means the formation of covalent linkages.

*"Delayed union"* as generally used herein means a bone fracture that has not healed within 3-4 months, i.e. a time span that is considered adequate for normal bone healing. Delayed union suggests that union is slow but will eventually occur without additional surgical or non-surgical intervention. However, a delayed union may, in some cases, progress to a non-union at a later timepoint.

*"Fibrin matrix"* as generally used herein means the product of a process in which substantially all of the precursor components, fibrinogen and thrombin, crosslink in the presence of a calcium source and Factor XIIIa to form a three-dimensional network.

*"Matrix"* as generally used herein refers to a material intended to interface with biological systems to treat, augment, or replace any tissue or function of the tissue depending on the material either permanently or temporarily. The matrix can serve as a delivery device for PTH incorporated therein and/or as a cell-ingrowth matrix. The matrices described herein are formed from liquid precursor components which are able to form a sca ffold in the body at the site of need. The terms "matrix", "gel" or biomaterials are used synonymously herein. The terms "matrix" and "gel" refer to the composition formed after the precursor components are mixed together. Thus the terms "matrix" and "gel" encompass partially or fully crosslinked polymeric networks. They may be in the form of a liquid, semi-solid, such as a paste, or a solid. Depending on the type of precursor materials, the matrix may be swollen with water but not dissolved in water, i.e. form a hydrogel which stays in the body for a certain period of time.

*"Naturally occurring precursor components or polymers"* as generally used herein refers to molecules which could be found in nature.

*"Non-union"* as generally used herein means a bone fracture, that does not show progression of fracture healing within 3 to 6 months following injury (depending on the type and location of fracture) in monthly radiographic studies.

*"PTH"* as used herein includes the human sequence of PTH₁₋₈₄ and all truncated, modified and allelic versions of PTH which exhibit bone formation properties, in particular when incorporated preferably covalently bound to a fibrin matrix. Preferred truncated versions of PTH are PTH₁₋₃₈, PTH₁₋₃₄, PTH₁₋₃₁ or PTH₁₋₂₅. Most preferred is PTH₁₋₃₄. Preferably, the PTH is human PTH, although PTH from other sources, such as bovine PTH, may be suitable.

*"PTH fusion peptide*" as generally used herein refers to a peptide which contains at least a first and a second domain. One domain contains a PTH, preferably PTH 1-34 and the other domain contains a substrate domain crosslinkable to a fibrin matrix during or after its formation. An enzymatic or hydrolytic degradation site can also be present between the first and the second domain.

*"Periosteum"* as used herein means the outer layer of bones forming a dense, fibrous layer, with the exception of those portions that form a joint structure which covers the entire bone structure and contains the vasculature that nourishes the exterior bone tissue.

*"Physiological"* as generally used herein means conditions as they can be found in living vertebrates. In particular, physiological conditions refer to the conditions in the human body such as temperature and pH. Physiological temperatures mean in particular a temperature range of between 35°C to 42°C, preferably around 37°C.

*"Repair or healing of bone fractures"* as generally used herein means rejoining and realigning the ends of broken bones.

*"Supplemented Matrices* or biomaterial as generally used herein means a matrix having incorporated PTH.

### I. Supplemented Matrices

### A. Matrix Materials

For tissue repair or regeneration, cells must migrate into a wound bed, proliferate, express matrix components or form extracellular matrix, and form a final tissue shape. Multiple cell populations must often participate in this morphogenetic response, frequently including vascular and nerve cells. Matrices have been demonstrated to greatly enhance, and in some cases have been found to be essential, for this to occur.

Approaches have been made in developing matrices from natural or synthetic origins or a mixture of both. The fibrin matrix, which is described in WO 03/052091, has been found to be suitable a matrix material for the repair of bone fractures.

The matrix is formed by crosslinking ionically, covalently, or by combinations thereof precursor molecules and/or by swelling one or more polymeric materials, i.e. matrices, to form a polymeric network having sufficient inter-polymer spacing to allow for in-growth or migration into the matrix of cells. In one embodiment the matrix is formed of proteins, preferably proteins naturally present in the patient into which the matrix is to be implanted. A particularly preferred matrix protein is fibrin, although matrices made from other proteins, such as collagen and gelatine can also be used. Polysaccharides and glycoproteins may also be used to form the matrix.

### Fibrin Matrices

Fibrin is a natural material which has been reported for several biomedical applications. Matrices made from fibrin have been described as material for cell in-growth matrices in U. S. Patent No. 6,331,422 to Hubbell et al*.* Fibrin has been used in sealants because of its ability to bind to many tissues and its natural role in wound healing. Some specific applications include use as a sealant for vascular graft attachment, heart valve attachment Additionally, these matrices have been used as drug delivery devices, and for neuronal regeneration. Although fibrin matrices provide a solid support for tissue regeneration and cell in-growth, there are few active sequences in the monomer that directly enhance these processes.

The process by which fibrinogen is polymerized into fibrin has also been characterized. Initially, a protease cleaves the dimeric fibrinogen molecule at the two symmetric sites. There are several possible proteases than can cleave fibrinogen, including thrombin, peptidase, and protease III, and each one severs the protein at a different site. Once the fibrinogen is cleaved, a self-polymerization step occurs in which the fibrinogen monomers come together and form a non-covalently crosslinked polymer gel. This self-assembly happens because binding sites become exposed after protease cleavage occurs. Once they are exposed, these binding sites in the centre of the molecule can bind to other sites on the fibrinogen chains, which are present at the ends of the peptide chains. In this manner, a polymer network is formed. Factor XIIIa, a transglutaminase activated from Factor XIIIa by thrombin proteolysis, may then covalently crosslink the polymer network. Other transglutaminases exist and may also be involved in covalent crosslinking and grafting to the fibrin network.

Once a crosslinked fibrin matrix is formed, the subsequent degradation is tightly controlled. One of the key molecules in controlling the degradation of fibrin is α2-plasmin inhibitor. This molecule acts by crosslinking to the α chain of fibrin through the action of Factor XIIIa. By attaching itself to the matrix, a high concentration of inhibitor can be localized to the matrix. The inhibitor then acts by preventing the binding of plasminogen to fibrin and inactivating plasmin. The α2-plasmin inhibitor contains a glutamine substrate. The exact sequence has been identified as NQEQVSPL (SEQ ID NO: 2), with the first glutamine being the active amino acid for crosslinking.

It has been demonstrated that bi-domain peptides, which contain a factor XIIIa substrate sequence and a bioactive peptide sequence, can be cross-linked into fibrin matrix and that this bioactive peptide retains its cellular activity *in vitro.*

Depending on the indication and substances mixed into the fibrin matrix the concentration of thrombin might vary. In one preferred embodiment, the fibrin matrix contains fibrinogen in a range of 5 to 65 mg per millilitre fibrin matrix, more preferably 15 to 60 mg per millilitre fibrin matrix, even more preferably from 25 to 55 mg per millilitre fibrin matrix, and most preferably 30 to 45 mg per millilitre fibrin matrix. Thrombin is present in a range of 0.5 to 5 I.U. per millilitre fibrin matrix, more preferably in a range of between 1.25 to 3.25 I.U. per millilitre fibrin matrix, most preferably from 1.5 to 2.5 I.U. per millilitre fibrin matrix. Additionally a calcium ion source helps to form the fibrin matrix. The calcium ion source is preferably CaCl₂ * 2H₂O in a concentration range from 0.5 to 5 mg per ml fibrin matrix, even more preferably in a concentration from 2 to 3.5 mg per ml fibrin matrix, most preferably in a concentration range from 2.5 to 3 mg per ml fibrin matrix. This composition does not take into account any water used to wet any potential granules before mixing into fibrin matrix since said water stays in the pores of the granules throughout the process of matrix formation and thus does will not have any dilutive effect on the fibrinogen and thrombin concentration in the fibrin matrix. I.U. stands for one international unit of thrombin and is defined as the activity contained in 0.0853 mg of the First International Standard of Human Thrombin.

### Precursor components for forming fibrin matrices

The fibrin matrix is preferably formed from two precursor components which can be in the form of solutions. The first precursor component, typically in form of a solution, contains fibrinogen, preferably in a concentration range from 10 to 130 mg fibrinogen per millilitre precursor solution, more preferably from 30 to 120 mg fibrinogen per millilitre precursor solution, even more preferably from 50 to 110 mg fibrinogen per millilitre precursor solution, and most preferably from 60 to 90 mg fibrinogen per millilitre precursor solution. If thrombin has to be added to form the matrix, the second precursor component, also typically in form of a solution, contains thrombin, preferably in a concentration range from 1 to 10 I.U. thrombin per millilitre precursor solution, more preferably from 2.5 to 6.5 I.U. thrombin per millilitre precursor solution, most preferably from 3 to 5 I.U. thrombin per millilitre precursor solution. Additionally a calcium ion source is in one of the precursor solutions. The calcium ion source is preferably CaCl₂ * 2H₂O in a concentration range from 1 to 10 mg per ml precursor solution, even more preferably from 4 to 7 mg per ml precursor solution, most preferably from 5 to 6 mg per ml precursor solution. Optionally, an enzyme capable of catalyzing the matrix formation, like Factor XIIIa, is added to a precursor solution. Preferably, Factor XIIIa is present in a concentration range from 0.5 to 100 I.U. per millilitre precursor solution, more preferably from 1 to 60 I.U. per millilitre precursor solution, and most preferably from 1 to 10 I.U. per millilitre precursor solution.

### B. Cell attachment sites

Cells interact with their environment through protein-protein, protein-oligosaccharide and protein-polysaccharide interactions at the cell surface. Extracellular matrix proteins provide a host ofbioactive signals to the cell. This dense network is required to support the cells, and many proteins in the matrix have been shown to control cell adhesion, spreading, migration and differentiation. Some of the specific proteins that have been shown to be particularly active include laminin, vitronectin, fibronectin, fibrin, fibrinogen and collagen. Many studies of laminin have been conducted, and it has been shown that laminin plays a vital role in the development and regeneration of nerves *in vivo* and nerve cells *in vitro,* as well as in angiogenesis. Some of the specific sequences that directly interact with cellular receptors and cause either adhesion, spreading or signal transduction have been identified.

Laminin, a large multidomain protein, has been shown to consist of three chains with several receptor-binding domains. These receptor-binding domains include the YIGSR (SEQ ID NO: 1) sequence of the laminin B1 chain, LRGDN (SEQ ID NO: 3) of the laminin A chain and PDGSR (SEQ ID NO: 4) of the laminin B1 chain. Several other recognition sequences for cells have also been identified. These include IKVAV (SEQ ID NO: 5) of the laminin A chain and the sequence RNIAEIIKDI (SEQ ID NO: 6) of the laminin B2 chain. Particularly preferred is the RGD sequence from fibronectin.

In a further preferred embodiment peptide sites for cell adhesion are incorporated into the matrix, namely peptides that bind to adhesion-promoting receptors on the surfaces of cells. Such adhesion promoting peptides include those described above. Particularly preferred are the RGD sequence from fibronectin and the YIGSR (SEQ ID NO: 1) sequence from laminin. Cell attachment sites can be included with some of the natural matrices. The incorporation can be accomplished, for example, by mixing a cysteine-containing cell attachment peptide with the precursor molecule including the conjugated unsaturated group, such as PEG acrylate, PEG acrylamide or PEG vinylsulfone. This step may occur shortly, e.g. a few minutes, before mixing with the remainder of the precursor component including the nucleophilic group, such as thiol-containing precursor component. If the cell attachment site does not include a cysteine, it can be chemically synthesized to include one. During this step, the adhesion-promoting peptide will become incorporated into one end of the precursor multiply functionalized with a conjugated unsaturation; when the remaining multi-thiol is added to the system, a cross-linked network will form.

The concentration of adhesion sites covalently bound into the matrix can influences the rate of cell infiltration. For example, for a given hydrogel, a RGD concentration range can be incorporated into the matrix with supports cell in-growth and cell migration in an optimal way. The optimal concentration range of adhesion sites like RGD is between 0.04 and 0.05 mM and even more preferably 0.05 mM in particular for a matrix having a water content between equilibrium concentration and 92 weight % after termination of water uptake.

### C. PTH

The term "PTH" as used herein includes the human sequence of PTH₁₋₈₄ and all truncated, modified and allelic versions of PTH which exhibit bone formation properties when covalently bound to biodegradable natural or synthetic matrices. Preferred truncated versions of PTH are PTH₁₋₃₈, PTH₁₋₃₄, PTH₁₋₃₁, PTH₁₋₂₈ or PTH₁₋₂₅. Most preferred is PTH₁₋₃₄. Preferably, the PTH is human PTH, although PTH from other sources, such as bovine PTH, may be suitable. PTH in a range of between 0.01 to 2 mg PTH/mL matrix, is suitable for the repair and healing of bone fractures with the proviso that a concentration of 0.4mg PTH/mL matrix is not included. Preferably the concentration of PTH is in a range of between 0.1 to 1.7 mg/mL matrix, even more preferably the concentration range is in a range of between 0.3 to 1.5mg/mL matrix and most preferably in a concentration range of between 0.4 to 1.1 mg/mL matrix with the proviso that a concentration of 0.4mg PTH/mL of matrix is not included. In a preferred embodiment the matrix is a fibrin matrix.

### D. PTH Fusion Peptides

In a preferred embodiment the PTH is a PTH fusion peptide, which comprises at least two domains wherein the first domain comprises PTH and the second domain comprises a crosslinkable substrate domain crosslinkable to the matrix during or after its formation. The substrate domain is a domain for an enzyme, preferably a substrate domain for a transglutaminase ("transglutaminase substrate domain"), more preferably for a tissue transglutaminase ("tissue transglutaminase substrate domain") and most preferred it is a substrate domain for Factor XIIIa ("Factor XIIIa substrate domain"). Transglutaminases catalyse acyl-transfer reactions between the gamma-carboxamide group of protein bound glutaminyl residues and the epsilon- amino group of lysine residues, resulting in the formation of N-epsilon-(gamma-glutamyl)lysine isopeptide side chains bridges. The amino acid sequence of the PTH fusion peptide can be designed to further contain an enzymatic or hydrolytic cleavage site, thus that the PTH can be released with little or no modification to the primary structure.

Transglutaminase substrate domains and in particular, Factor XIIIa substrate domains, are suitable to link the fusion peptide to natural matrices, such as fibrin matrices. When used with a fibrin matrix the degradation site in the fusion peptide is preferably enzymatically degradable, so that the release of the PTH is controlled by cell specific processes, such as localized proteolysis.

The crosslinkable substrate domain may include *GAKDV* (SEQ ID NO: 7), KKKK (SEQ ID NO: *8),* YRGD*TIGEGQQHHLGG* (SEQ ID NO: 9), or *NQEQVSPL* (SEQ ID NO: 2).

The most preferred Factor XIIIa substrate domain has an amino acid sequence of *NQEQVSPL* (SEQ ID NO: 2) and is herein referred to as "TG" and TG-PTH.

The PTH fusion peptide may be produced recombinantly or by chemical synthesis. The PTH 1-34 fusion peptide is preferably produced by chemical synthesis.

Transglutaminase substrate domains suitable for the purposes of the present invention have been described in detail including their amino acid sequences in WO 03/052091, the respective content thereof being incorporated herein by reference.

As described herein as a preferred embodiment the Factor XIIIa substrate domain is either directly linked to the PTH₁₋₃₄ or it can include a degradation site between the PTH (first domain) and the NQEQVSP (SEQ ID NO: 2) sequence (second domain). As such, the PTH₁₋₃₄ fusion peptide may be incorporated within fibrin during coagulation via a factor XIIIa substrate and released as PTH₁₋₃₄.

The degradation sites allow the PTH to be released with little or no modification to the primary peptide sequence, which may result in higher activity of the factor. In addition, it allows the release of the factor to be controlled by cell specific processes. This allows factors to be released at different rates within the same material depending on the location of cells within the material. This also reduces the amount of total PTH₁₋₃₄ needed, since its release is controlled by cellular processes. In one possible explanation for the strong healing of the above mentioned bone defects with PTH incorporated and preferably bound to a matrix, it is deemed important that the PTH is administered locally over an extended period of time (i.e. not just a single pulsed dose) but not in a continuous fashion. This is accomplished by a slow degradation, through either enzymatic cleavage or hydrolytic cleavage of the matrix. In this way, the molecule is then delivered through a pseudo-pulsed effect that occurs over a sustained period of time. When a preosteoblastic cell infiltrates the matrix, it will encounter a PTH molecule which will induce further proliferation of the preosteoblast as well as synthesis of multiple growth factors crucial for new bone formation. However, if that particular cell does not continue to liberate bound PTH from the matrix, it will not begin to produce interleukin-6, thereby avoiding the later stage catabolic effects on osteoclasts formation. The net result is then higher bone mineral density and net formation of bone matrix. Finally, the therapeutic effects of the peptide are localized to the defect region and are subsequently magnified.

### Degradation sites of the fusion peptide

An enzymatic or hydrolytic degradation site can be present between the first and the second domains of the fusion peptide. The degradation sites may be degradable by specific enzymatic degradation. This allows the release of the PTH to be controlled by cell specific processes, such as localized proteolysis. It allows PTH to be released at different rates within the matrix depending on the location of cells within the material. Preferably the degradation site is cleavable by an enzyme selected from the group consisting of plasmin and matrix metallo-proteinase. By careful selection of Kₘ and k_{cat} of this enzymatic degradation site, degradation could be controlled to occur either before or after the fibrin matrix and/or by utilizing similar or dissimilar enzymes to degrade the matrix. These degradable sites allow the engineering of more specific release of PTH from fibrin matrices. The degradable site can be cleaved by enzymes released from cells which invaded the matrix. The degradation site allows the rate of delivery to be varied at different locations within the matrix depending on cellular activity at that location and/or within the matrix. Additional benefits include the lower total drug dose within the delivery system, and spatial regulation of release which permits a greater percentage of the drug to be released at the time of greatest cellular activity. The degradation site is abbreviated "pl" in the context of the present invention.

Proteolytically degradable sites could include substrates for collagenase, plasmin, elastase, stromelysin, or plasminogen activators. Exemplary substrates are listed below. N1-N5 denote amino acids 1-5 positions toward the amino terminus of the protein from the site were proteolysis occurs. N1'-N4' denote amino acids 1-4 positions toward the carboxy terminus of the protein from the site where proteolysis occurs.

**Table 1: Sample substrate sequences for protease**

| **Protease** | **N5** | **N4** | **N3** | **N2** | **N1** | **N1'** | **N2'** | **N3'** | **N4'** | **SEQ ID NO:** |
|---|---|---|---|---|---|---|---|---|---|---|
| Plasmin¹ | | | L | I | K | M | K | P | | SEQ ID NO: 10 |
| Plasmin¹ | | | N | F | K | S | Q | L | | SEQ ID NO: 11 |
| Stromelysin² | Ac | G | P | L | A | L | T | A | L | SEQ ID NO: 12 |
| Stromelysin² | | Ac | P | F | E | L | R | A | NH₂ | SEQ ID NO: 13 |
| Elastase³ | | | Z- | A | A | F | A | NH₂ | | SEQ ID NO: 14 |
| Collagenase⁴ | | G | P | L | G | I | A | G | P | SEQ ID NO: 15 |
| t-PA⁵ | P | H | Y | G | R | S | G | G | | SEQ ID NO: 16 |
| u-PA⁵ | P | G | S | G | R | S | A | S | G | SEQ ID NO: 17 |

References:
1. Takagi and Doolittle, (1975) Biochem. 14:5149-5156.
2. Smith et al., (1995). J. Biol. Chem. 270:6440-6449.
3. Besson et al., (1996) Analytical Biochemistry 237:216-223.
4. 4. Netzel-Arnett et al., (1991) J. Biol. Chem.. 266:6747-6755.
5. Coombs et al., 1998. J. Biol. Chem. 273:4323-4328.

A preferred embodiment is the sequence YKNR (SEQ.NO. 18) between the first domain and the second domain makes the linkage plasmin degradable.

A particular preferred PTH fusion peptide is TGplPTH:
NQEQVSPLYKNRSVSEIQLMHNLGKHLNSMERVEWLRKKLQDVHNF (SEQ ID NO:19)

Preferred fusion proteins include:
TG-PTH₁₋₃₄: This is a modified form of PTH comprising the amino acids 1-34 of the native PTH as well as a TG (transglutaminase) substrate domain:
NQEQVSPLSVSEIQLMHNLGKHLNSMERVEWLRKKLQDVHNF (SEQ ID NO: 20)
TG-pl-PTH₁₋₃₄: This form corresponds to TG-PTH except that it additionally contains a plasmin-degradable sequence (pl) between the TG sequence and the PTH₁₋₃₄:
NQEQVSPLYKNRSVSEIQLMHNLGKHLNSMERVEWLRKKLQDVHNF (SEQ ID NO: 19)

Enzymes that could be used for proteolytic degradation are numerous. Proteolytically degradable sites could include substrates for collagenase, plasmin, elastase, stromelysin, or plasminogen activators.

In another preferred embodiment an oligo-ester domain could be inserted between the first and the second domain. This can be accomplished using an oligo-ester such as oligomers of lactic acid.

### Combination of Matrices or precursor components and PTH

PTH or PTH fusion peptide is preferably in a range of between 0.01 to 2 mg PTH/mL matrix or precursor components forming the matrix, which is suitable for the repair and healing of bone fractures with the proviso that a concentration of 0.4 mg PTH/mL fibrin matrix or precursor components forming the matrix is not included. Preferably the concentration of PTH is in a range of between 0.1 to 1.7 mg/mL fibrin matrix or precursor components forming the matrix, even more preferably the concentration range is in a range of between 0.3 to 1.5mg/mL fibrin matrix or precursor components forming the matrix and most preferably in a concentration range of between 0.4 to 1.1 mg/mL fibrin matrix or precursor components forming the matrix with the proviso that a concentration of 0.4mg PTH/mL of fibrin matrix or precursor components forming the matrix is not included. Depending on the age of the patient, certain concentrations of the PTH or PTH fusion peptide in the matrices are preferred. If the formulation is applied to bone fractures in children, the concentration of PTH or PTH fusion peptide is preferably below 0.4 mg PTH or PTH fusion peptide/mL matrix or precursor components forming the matrix but above 0.01 mg PTH or PTH fusion peptide/mL matrix or precursor components forming the matrix. Specifically the concentration of PTH or PTH fusion peptide is in a range of between 0.01 and 0.35 mg PTH or PTH fusion peptide/mL matrix or precursor components forming the matrix, more preferably in a concentration range of between 0.1 to 0.3 mg/mL fibrin matrix or precursor components forming the matrix and most preferably in a concentration range of between 0.05 and 0.15 mg PTH or PTH fusion peptide/mL matrix or precursor components forming the matrix. Whereas if the formulation is applied to adults, the concentration of PTH or PTH fusion peptide is preferably above 0.4 mg PTH or PTH fusion peptide/mL matrix or precursor components forming the matrix but not higher than 2mg PTH or PTH fusion peptide/mL matrix or precursor components forming the matrix. Specifically the preferred concentration of PTH or PTH fusion peptide is in a range of between 0.45 and 2 mg PTH or PTH fusion peptide/mL matrix or precursor components forming the matrix, more preferably between 0.7 and 1.5 mg PTH or PTH fusion peptide/mL matrix or precursor components and most preferably between 0.9 and 1.1 mg PTH or PTH fusion peptide/mL matrix or precursor components forming the matrix. In a preferred embodiment the matrix is a fibrin matrix.

### E. Granular material

The matrix may also contain a granular material, preferably the granular material contains a calcium mineral. his granular material mainly supports the mechanical properties of the fibrin matrix to adapt the matrix to the specific needs of the indication. The granular material can be any biocompatible material providing the necessary mechanical support to the composition, whereby the degree of mechanical support is dependent on the indication. Preferably the granular material is a ceramic compound. Biodegradable ceramic compounds have shown favourable properties in the matrix. The ceramic compound preferably comprises a calcium mineral, like hydroxyapatite, calcium phosphate or calcium sulphate. Suitable materials include biodegradable porous mixtures of hydroxyapatite and tricalciumphosphate, like TRICOS® from Biomatlante (France) or CAMCERAM® from Cam Implants, Leiden (Netherlands). Most preferred is a degradable mixture of hydroxyapatite and tricalciumphosphate . One example is a product marketed under the tradename TRICOS®, which contains a mixture of 60% hydroxyapatite and 40% tricalciumphosphate Another porous hydroxyapatite/tricalcium phosphate granules, is CAMCERAM®,

It is also possible to use nonporous hydroxyapatite/tricalcium phosphate granules, pure hydroxyapatite granules (porous or nonporous), tricalcium phosphate granules (porous or nonporous), calcium sulfate granules, bone chips (either autograft or allograft) or xenograft bone chips.

The amount of granules in the supplemented matrix is regulated by the dead space of the granules. In a preferred embodiment, the ratio is 1:1 of dead space volume in the granules to total volume of liquids in the matrix; this would constitute the upper limit of granules in the matrix. Dependent on the kind of fracture any amount of granules less than that are possible if the kind of bone fracture requires only a minor mechanical support.

### II. Methods of Application

The supplemented matrix may be formed *in situ* at the site of need in or on the body or may be preformed and then implanted into the desired location, i.e. at the fracture site. The *in situ* gelling/formation can occur by mixing fibrin precursor solutions containing the PTH with the granules, if present, and applying the matrix to the fracture site. In another embodiment the matrix can be formed outside the body and then applied in the preformed shape. When a fibrin matrix is being formed the precursor components should be kept separate prior to application to prevent premature polymerization. To prevent premature contact prior to administration, a kit which separates the precursor solutions from each other may be used. Upon mixing under conditions that allow polymerization, the precursor solutions form a PTH supplemented three dimensional network. Depending on the precursor components and their concentrations, the gelling time can be tailored to the need.

In one embodiment the matrix is formed from fibrinogen. Fibrinogen, through a cascade of various reactions gels to form a matrix, when brought in contact with thrombin and a calcium source at appropriate temperature and pH. The three components, fibrinogen, thrombin, and the calcium source, should be stored separately. However, as long as at least one of the three components is kept separated the other two components can be combined prior to administration.

The supplemented matrix should be designed such that the introduction of the matrix is possible as liquids or in a paste-like consistence which permeate and fill the bone fracture site. Solidification allows the scaffold, and therefore the active agent, to be retained at the fracture site.

Cells can also be added to the supplemented matrix prior to or at the time of implantation, or even subsequent to implantation, either at or subsequent to crosslinking of the polymer to form the matrix. This may be in addition to or in place of crosslinking the matrix to produce interstitial spacing designed to promote cell proliferation or in-growth.

In one embodiment fibrinogen is dissolved (which may contain additionally aprotinin to increase stability) in a buffer solution at physiological pH (in a range from pH 6.5 to 8.0, preferably from pH 7.0 to 7.5) and stored separately from a solution of thrombin in a calcium chloride buffer. The buffer solution for the fibrinogen can be a histidine buffer solution including additionally NaCl or TRIS buffer saline. Both solutions are typically stored frozen and have to be thawed prior to application.

In a preferred embodiment, a kit, which contains PTH, preferably a PTH fusion protein, a granular material preferably containing a calcium mineral, fibrinogen, thrombin, and a calcium source, is provided. Optionally, the kit contains a crosslinking enzyme, such as Factor XIIIa. The PTH, preferably the PTH fusion protein, may be present in either the fibrinogen or the thrombin solution. In a preferred embodiment the fibrinogen solution contains the PTH, preferably a PTH fusion peptide.

The fibrinogen and thrombin solutions are preferably mixed by a two way syringe device, in which mixing occurs by squeezing the contents of both syringes through a mixing chamber and/or needle and/or static mixer.

In a preferred embodiment both fibrinogen and thrombin are stored separately in lyophilised form. Either of the two precursor components can contain the fusion protein and the granular material. Prior to use, a tris or histidine buffer is added to the fibrinogen, the buffer may additionally contain aprotinin to form a fibrinogen precursor solution. Prior to use, the lyophilized thrombin is dissolved in the calcium chloride solution to form a thrombin precursor solution. Subsequently, the fibrinogen and the thrombin precursor solutions are placed in separate containers, vials or syringe bodies and mixed using a two-way connecting device, such as a two-way syringe. Optionally, the containers, vials or syringe bodies are bipartited, thus having two chambers separated by an adjustable partition which is perpendicular to the container, vial or syringe body wall. One of the chambers contains the lyophilised fibrinogen or thrombin, while the other chamber contains an appropriate buffer solution. When the plunger is pressed down, the partition moves and releases the buffer into the fibrinogen chamber to dissolve the fibrinogen. Once both fibrinogen and thrombin are dissolved, both bipartite syringe bodies are attached to a two way connecting device and the contents are mixed by squeezing them through the injection needle attached to the connecting device. Optionally, the connecting device contains a static mixer to improve mixing of the contents.

The granular material may be provided in a separate syringe. According to a preferred embodiment, the granular material is wetted by injecting sterile water into the syringe. Subsequently, the above two-way syringe containing the fibrin precursor solutions and the PTH is attached to the syringe containing the wetted granular material. The entire content of the two-way syringe is transferred into the syringe containing the granular material. The entire content is subsequently injected to the site of the bone fracture and is moldable for several minutes..

Additionally, other components beside the above-mentioned ingredients may be incorporated into the precursor solutions and/or resulting matrices. For example, a material containing a calcium mineral, i.e. a naturally occurring homogenous substance containing calcium ions such as hydroxyapatite, may be used.

While the compositions and methods have been described in terms of preferred embodiments, it will be apparent to those of skill in the art that variations may be applied to the composition, methods and in the steps or in the sequence of steps of the method described herein without departing from the concept, spirit and scope of the invention.

### Examples

### Example 1: Bioactivity of PTH₁₋₃₄ and TGpIPTH₁₋₃₄

PTH₁₋₃₄- peptide showing similar activity to the full length PTH₁₋₈₄, and proteins of this length can be synthesized by standard solid state peptide synthesis methods.

All peptides were synthesized on solid resin using an automated peptide synthesizer using standard 9-fluorenylmethyloxycarbonyl chemistry. Peptides were purified by c18 chromatography and analyzed using reverse phase chromatography via HPLC to determine purity as well as mass spectroscopy (MALDI) to identify the molecular weight of each product. Using this method, PTH₁₋₃₄as well as, TG-pl-PTH₁₋₃₄NQEQVPLYKNRSVSEIQLMHNLGKHLNSMERVEWL-RKKLQDVHNF (SEQ ID NO. 19) and TGPTH₁₋₃₄ NQEQVPLS-VSEIQLMHNLGKHLNSMERVEWLRKKLQDVHNF (SEQ ID NO. 20 ) were synthesized. TGplPTH₁₋₃₄ and TGPTH₁₋₃₄ differs from PTH₁₋₃₄ in that it additionally comprises the Factor XIIIa substrate domain which is linked to PTH₁₋₃₄ via the plasmin degradable pl-sequence YKNR (SEQ ID NO. 18) in case of TGplPTH₁₋₃₄ and directly in case of TGPTH₁₋₃₄.

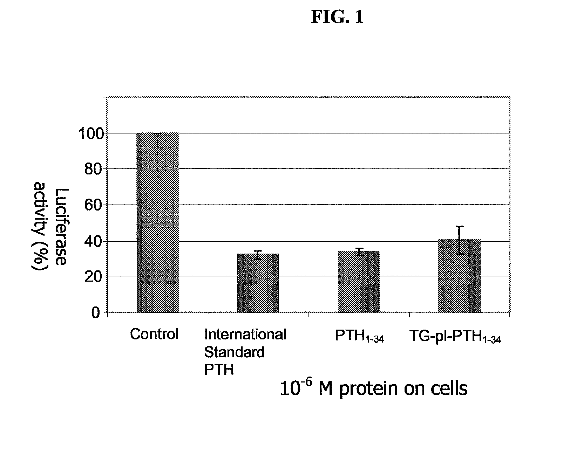

To study the bioactivity of the PTH fusion peptides, a reporter gene assay was established. In this assay, a plasmid that contains luciferase reporter gene which is linked to the promoter for the parathyroid hormone receptor is transfected into cells. Then, if the cell is exposed to PTH and the PTH subsequently binds to its receptor on the cell, a signal cascade, directed through elevated cAMP levels, is initiated. Through a natural feedback regulation, this then leads to a reduction of PTH receptor levels. As the reduction is directed through the promoter, it also then leads to a decrease in production of the linked reporter gene. Using this assay, the activity of both native PTH₁₋₃₄ as well as TG-pl-PTH1-34 were studied and compared to an international standard. It was observed that both of these molecules showed a similar level of activity, as the reduction in reporter gene expression for both was the same, and this level of activity was the same as for the international standard. The results are shown in Figure 1.

### Example 2: PTH release from a fibrin matrix

A fibrin matrix was made from TISSEEL^{®} Kit (Baxter AG, CH-8604 Volketswil/ZH) fibrin precursor components. The composition is listed in Table 2. In the presence of 0,1 µg/ml of PTH₁₋₃₄ or TGPTH₁₋₃₄ was then added to the thrombin, and mixed to form a homogenous concentration. TGPTH₁₋₃₄ only has a transglutaminase sequence at the amino terminus, without a degradation site. Thus, TGPTH₁₋₃₄ can only be liberated by degradation of the fibrin matrix itself. This peptide was synthesized as described above in Example 1.

For the first release assay a fibrin matrix of 50 µl with 0.1 mg PTH or TGPTH per ml fibrin matrix was incubated at 37°C in 10 ml buffer. Therefore, the concentration of PTH or TGPTH in the buffer in case of a total release would be 0.5 µg PTH or TGPTH /mL fibrin matrix. In order to compare the stability of PTH or TGPTH during the assay, samples of PTH or TGPTH were diluted directly in the buffer to a concentration of 0.5 µg PTH or TGPTH /mL fibrin matrix. Different buffers were tested: distilled water, phosphate buffer saline, tris-buffer saline.

Aliquots were taken at days 0, 1, 2, 4 and 6 and analysed by direct ELISA. The results showed that the PTH was not stable for more than 2 days in any of the buffers. Therefore, no conclusion could be made on the release data. The PTH stability was certainly affected by its low concentration and the buffers that were not optimal.

The release experiment was repeated by using a stabilizing buffer containing 50 mM mannitol in a 10mM sodium acetate buffer. In addition, the buffer was exchanged every 2 days in order to prevent any degradation of peptide. The concentration of PTH or TGPTH was increased to 1 mg PTH or TGPTH /mL fibrin matrix in a 100 µl fibrin matrix and the incubation was achieved in 1 ml buffer. The concentration of PTH or TGPTH in the buffer in case of a total release would be 100 µg/mL fibrin matrix (200 times more than before). As in the first experiment, spiked samples (same amount of PTH or TGPTH dissolved in the buffer as control) were prepared to evaluate the stability of PTH or TGPTH during the experiment (100 µg/ml). Samples were collected every 2 to 4 days (with a change of buffer) during 2 weeks and analysed by direct ELISA. Spiked samples were also collected every 2 days. The results showed that under these conditions PTH and TGPTH are stable over 2 weeks.

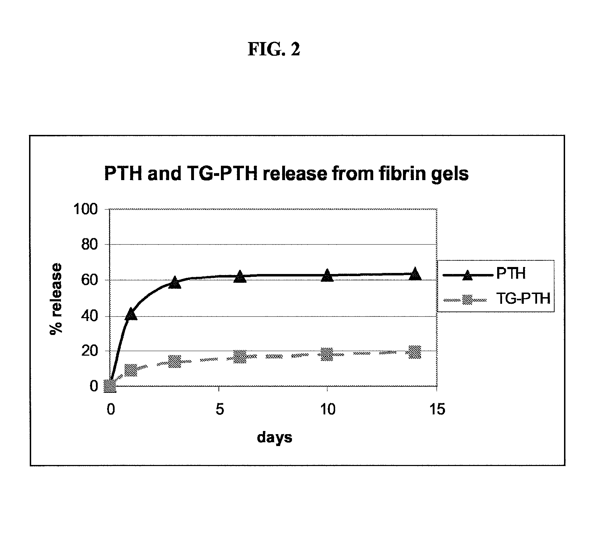

As can be seen from Figure 2, the major release from the fibrin matrix is achieved within 3 days. Almost 60% of PTH and 13% of TGPTH were released after day 3. These data demonstrate the retention of PTH in the fibrin matrix is highly enhanced by addition of the TG sequence.

### Example 3: Sheep Tibial Defect Model

The fibrin matrix was formed starting from the TISSEEL^{®} Kit (Baxter AG, CH-8604 Volketswil/ZH) giving 4mL fibrin matrix. TISSEEL^{®} is produced from human derived pooled plasma and the content of active ingredients may vary from lot to lot within predefined ranges.

*Matrix:* The matrix was prepared using 3 separate syringes, a two-way syringe containing fibrin precursor solution and PTH fusion peptide (syringes 1 and 2) and two one-way syringes containing granules (syringes 3 and 4). Table 2 lists the final composition used.

**Table 2: Final Composition comprising TISSEEL^{®} and active component**

| **Ingredients** | | | **Dose per 2 mL gel** |
|---|---|---|---|
| ***Syringe 1 (1 mL)*** | | | |
| | Active Component: | | 0.4-10 mg |
| | PTH₁₋₃₄ fusion peptide (TGpIPTH₁₋₃₄) | | |
| | Clotting Agents | | |
| | | Fibrinogen (Human) | 66-100 mg |
| | Other Proteins | | |
| | | Aprotinin (Bovine) | 2046-3409 KIU |
| | | Human Albumin | 9.1 - 18.2 mg |
| | Buffer Components | | |
| | | Niacinamide | 2.7 - 8.2 mg |
| | | L-Histidine | 9.1 - 22.7 mg |
| | | Sodium Citrate | 4.4-8.8 mg |
| | | Polysorbate 80 | 0.6-1.7 mg |
| | | Water for Injection | to 1 mL |

| ***Syringe 2 (1 mL)*** | | | |
|---|---|---|---|
| | Clotting Agents | | |
| | | Thrombin (Human) | 2.5-6.5 I.U. |
| | Buffer Components | | |
| | | Calcium Chloride | 5.88 ± 0.6 mg |
| | | Sodium Chloride | 3.5-5.5 mg |
| | | Human serum albumin | 45-55 mg |
| | | Water for Injection | to 1 mL |

| ***Syringe 3*** | | | |
|---|---|---|---|
| | Water for injection | | 1 mL |

| ***Syringe 4*** | | | |
|---|---|---|---|
| | Hydroxyapatite/calcium phosphate granules (TCP granules) | | 1.75-2 g |

First the hydroxyapatite/calciumphosphate granules in syringe 4 were wetted by injection of the water of syringe 3. The fibrin precursor solution of syringe 1 (fibrinogen and PTH suspended in a solution with aprotinin, a serine proteinase inhibitor which helps reduce fibrolysis to retain the integrity of the fibrin matrix) was mixed with the fibrin precursor solution of syringe 2 (thrombin in a calcium chloride solution). TGpIPTH₁₋₃₄ was formulated into the fibrinogen component to give a final concentration in the matrix varying from of 0.1mg/mL to 10mg/mL of the fibrin matrix and during the gelation process TGpIPTH₁₋₃₄ became crosslinked to the matrix. Fibrin precursor solutions also contained other components of fibrin matrix, such as plasma fibronectin, Factor XIIIa, plasminogen, and human albumin. When the precursor solutions are in equal volumes, a clotting process occurs to form fibrin. The clotting process takes place over several minutes which allows for the simultaneous injection of the mixed solutions into syringe 4, which contain the wetted granules. The matrix can then be introduced at the site of need where it solidifies. Enough matrix was placed in the defect to completely fill it.

*Animals:* A total of 42 Swiss Alpine, female sheep ranging between 44-83 kg (mean: 62.8 kg) and 2.25 - 4.75 years of age were chosen as experimental animals. Seven groups were formed with autografts as positive and fibrin matrix plus TCP granules as negative controls. The other groups consisted of the same fibrin matrix and TCP granules, but differed only in dosage of the TGplPTH₁₋₃₄.. Groups were followed for 12 weeks, when animals were sacrificed at a university-owned slaughter house. All animal experiments were conducted according to the Swiss laws of animal protection and welfare and were authorized by the local Ethical Committee and Veterinary Authorities.

*Surgery:* A medial approach to the tibia shaft was performed directly above the bone and extending from the distal aspect of the stifle to the hock joint, the medial fascia of the tibia was incised and the medial aspect of the tibia exposed without disturbing the periosteum. A 11 hole, 3.5mm broad dynamic compression plate (Synthes) was contoured to the shaft with the distal end of the plate ending above the tibiotarsal joint. The plate was fixed to the bone using eleven 3.5 mm screws in a neutral position and distributing five screw holes distally and six proximally to the planned defect. The screws and plate were removed. Thereafter, the defect was marked on the bone with a scalpel blade using a gage ensuring a standardized 1 cm defect between the 5^{th} and 6^{th} screw hole. With an oscillating saw, the defect was cut under constant irrigation and preservation of soft tissues. The 1 cm segment including the circumferential periosteum was carefully removed and local bleeding out of the bone marrow or local soft tissue was stopped applying pressure with a gauze. The plate was repositioned and all screws reinserted before the matrix was filled into the bone gap as described above. Care was taken to distribute the matrix equally underneath the plate, on the lateral and as well as the transcortex.

For the positive autograft control group, autogenic bone was harvested through a local incision directly above the iliac crest. The bone marrow of the ileum was opened using multiple 3.5mm drill holes. The cancellous bone was harvested using a curette and was immediately placed into the surgical defect at the tibia. After filling of the defects, the medial fascia was closed routinely and the skin stapled (Signet 35W®, Auto Sutures, Connecticut, USA). While the animals were still anaesthetized, radiographs were taken using medio-lateral and caudo-cranial views of the tibia. Full casts (Scotch Cast™Plus, Laboratoires 3M Santé, France) were applied involving the entire distal extremities extending up to the middle of the stifle joint at the level of the patella. The sole of the claws was left open to ensure weight bearing on the fracture site, but preventing torsional or shear forces through the immobilization of the tibial shaft through the cast.

Animals were recovered in a suspension system that prevented the animals from lying down and getting up in a hurry causing re-fracturing of the limb. The animals were kept in the suspension system for 4-5 weeks, where they could sleep, eat, defecate and urinate without interference. Thereafter, they were kept in small stalls in groups of 2-3 animals for the rest of the study period.

Cast changes were performed every 7-10 days or earlier, if animals showed problems with weight bearing, and were left in place for minimally 4 weeks but maximally 12 weeks. Follow-up radiographs through the casts were taken at 4 and 8 weeks. At the time of sacrifice (12 weeks), the radiographs were taken using a faxitron (HP Electronics) after the soft tissue, the plates and screws were removed.

*Macroscopical evaluation*: After sacrifice, the bridging of the gap was assessed macroscopically focusing on plate and fracture stability, signs of inflammation and periosteal callus formation. All bones were documented with a digital camera (Minilta, Dimage 7) Mechanical stability was tested manually only, but with caution to prevent tissue damage for future histological preparation.

*Radiological evaluation*: A semi-quantitative score system was developed to evaluate the radiographs. High scores were favourable for bone healing. The radiographs were evaluated through three independent reviewers, who were uninformed about groups and time points during the study. All radiographs were scored during one session and if scores differed, the mean scores were taken for statistical evaluation.

*Histology.* Briefly, the bone sections were cut such that the entire previous gap was enclosed and the cut was made proximally and distally from the first screw hole. After fixation in 4% paraformaldehyde for 3-4 weeks, the bone blocks were washed, dehydrated in an ascending series of alcohol, defatted in xylene and finally infiltrated in acrylic resin. After infiltration was complete, polymerization was performed in Teflon forms. Sections were cut in the middle and parallel to the long axis of the bone using an Exacta band saw. Before the cut sections were mounted on acropal plastic slides, microradiographs were taken with the faxitron using a special film (Kodak PPL-2). Then sections were ground and polished to a thickness of 30-40 µm. Surface staining with toludine blue allowed differentiating between old and new bone matrix and also TCP granules.

For the thin sections (5µm), the blocks were cut in smaller pieces containing at least one cortex and part of the periosteal and endosteal callus. Sections were mounted on positively charged, chromalaune covered glass slides, deplastified and stained with either toluidine blue or von Kossa silver staining counterstained with McNeal. In the latter, the mineralized bone matrix is stained black, whereas the uncalcified osteoid is stained blue-turquoise.

*Qualitative histological evaluation* was conducted by focusing on cell types, signs of inflammation and mechanisms of degradation of the matrices.

*Semi-quantitative* evaluation concentrated on appearance of cell types using a specially developed score system, and histomorphometrical measurements served as basis for *quantitative* evaluation.

The regenerative capacity of the matrix (containing different concentrations of TGplPTH₁₋₃₄) was further tested in a segmental defect in the sheep. Here, unilateral 1 cm full thickness defects were created in the sheep tibiae. After the osteotomy, the periosteum was carefully removed to ensure that the defect was a non-union for the time period to be studied. After creation of the defect, an *in situ* gelating material was placed in the defect as described above in the methods of application section.

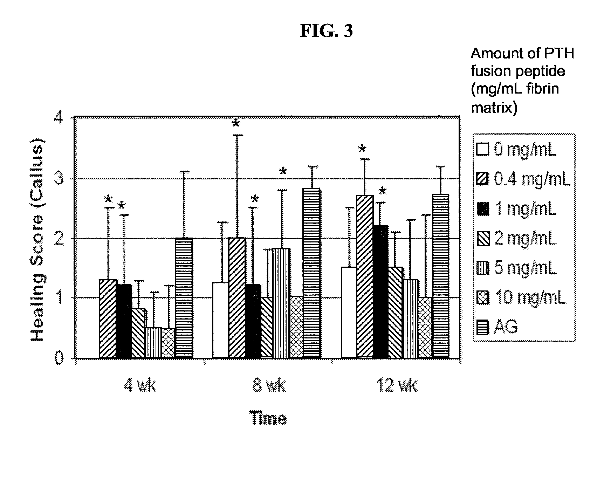

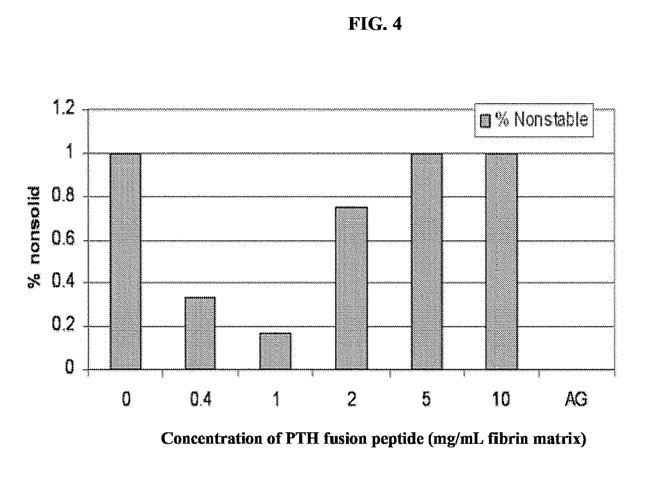

For comparative purposes, different doses of TG-PTH were tested: 0.4, 1, 2, 5 and 10 mg /ml, two control treatments were performed where no TGplPTH₁₋₃₄ was added to the fibrin -granule composite matrix (0 mg/ml) or an autograft (abbreviated "AG" in Figures 3 and 4) was applied (positive control).

X-ray photographies were taken every four weeks and the animals were sacrificed after twelve weeks. At each time point, the X-ray photographies were analyzed and the extent of healing was determined. Additionally, at the endpoint, the tibia was extracted for analysis via computer tomography (CT) as well as histology. Finally, as a full osteotomy was performed and then plated on one side, the defect was subject to significant stress and mechanical forces. If the material employed to treat the defect does not add significant strength, this can lead to bending of the plate and deformation of the angle. From the X-ray photographies and final samples, this is another parameter that has been measured.

The results of these experiments are shown in Figure 3:

When the fibrin plus TCP material alone (negative control) was tested in this model, a very low healing response was observed. Of the four animals treated with this control material, none of them showed clinically relevant healing, with one showing a moderate healing response and the three others showing classic signs of non-union after 12 weeks. Furthermore, within the first four weeks, all four of the animals showed significant bending of the plate and subsequent distortion of the defect.

In the next series of animals, the supplemented matrix was tested with 0.4, 1, 2, 5 or 10 mg/mL, of TGplPTH₁₋₃₄. As a first measure, these animals were observed with radiographic analysis to determine the amount of periosteal healing (callus formation). From the subsequent radiographs, it was observed that the treatment of these animals with various doses of TGplPTH₁₋₃₄ provided a dose dependent response over the range treated. Specifically, when the radiographs of these animals were examined, a much stronger periosteal healing response was observed at every time point for the doses between 0.4 and 2 mg/mL with 0.4 and 1 mg/mL providing statistically stronger periosteal healing when compared to those treated with the negative control material and healing that was equivalent to autograft as well at 12 weeks. Additionally, with the dose of 1 mg/mL, it was observed that most of the animals achieved a stable clinical union within 12 weeks (see Figure 4). In the group treated with 1 mg/mL TG-pl-PTH₁₋₃₄, after 4 weeks, the first sign of healing could already be observed. This healing was increased significantly at the 8 week time point, where already one animal showed clinical union, while the other five showed advanced healing. This then progressed such that after 12 weeks, 5 animals showed stable, clinical unions. Furthermore, a significant improvement in stability was observed, with only one animal showing bending of the plate, which occurred within the first four weeks.

In Figure 4, the stability of segmental tibial defects after 12 weeks is shown. After the sheep were sacrificed at the twelfth week, the plate was removed and the stability of the original gap was lightly determined. The percent of defects that were clearly unstable was then determined. It can be seen that none the animals that were treated with autograft had unstable defects, validating the model. Furthermore, when animals were treated with 1 mg/mL TGplPTH₁₋₃₄, only one animal had an unstable defect providing a similar strong healing response. Doses close to 1 mg/mL also showed some stability while higher doses and the zero dose showed a much lower stability at the endpoint.

As a final measure of healing, the rate of endosteal bone formation (formation of bone at the inner layer of long bones, i.e. inside the gap) and granule resorption was measured through histomorphometry. It was observed that with the 1 mg/mL TGplPTH₁₋₃₄ dose, the amount of endosteal bone formation was higher than with the other concentrations of TGplPTH_{1-34,} with higher doses providing lower amounts of new bone formation. Furthermore, it was observed that the rate of granule resorption was directly proportional to the rate of endosteal bone formation, with once again, the dose of 1 mg/mL TGplPTH₁₋₃₄ providing the best response (i.e. highest resorption rate). When the entire dose range was observed, it could be seen that higher doses provided lower rates of both endosteal bone formation and granule resorption, with the doses around 1 mg/mL TGplPTH₁₋₃₄ providing the best response.

The improved results in the group treated with TGplPTH₁₋₃₄ compared to the control groups demonstrated that the presence of clinically relevant doses of TGplPTH₁₋₃₄ in the supplemented matrices can lead to a strong improvement in healing. The faster healing, improved bone formation, as well as the improved stability in this highly mechanically stressed model are relevant characteristics to justify application of the supplemented matrix in distal radial compression fractures. Furthermore, in view of the results from the periosteal bone formation, endosteal bone formation and granule resorption and the overall stability, it could be seen that doses of between 0.4 mg/mL to 1.1 mg/mL TGplPTH₁₋₃₄ fibrin matrix is the most preferred concentration range.

The invention in particular pertains to the following embodiments:
1. A formulation comprising
   (i) a peptide selected from the group consisting of PTH and a PTH fusion peptide;
   (ii) a granular material comprising a calcium mineral and
   (iii) a composition capable of forming a fibrin matrix under physiological conditions comprising a fibrinogen precursor component and thrombin precursor component,
   wherein the PTH or PTH fusion peptide is present in a concentration range of between 0.01 to 2 mg/mL fibrin matrix or precursor components forming the matrix, with the proviso that a concentration of 0.4mg/mL fibrin matrix or precursor components forming the matrix is not included.
2. The formulation according to claim 1, wherein the fibrinogen precursor component or the thrombin precursor component further comprises a calcium source.
3. The formulation according to claim 1 or 2, wherein the PTH fusion peptide comprises at least two domains wherein the first domain comprises PTH and the second domain comprises a. crosslinkable substrate domain
4. The formulation according to any of claims 1 to 3, wherein the PTH is selected from the group consisting of PTH₁₋₈₄, PTH₁₋₃₈, PTH₁₋₃₄, PTH₁₋₃₁ and PTR₁₋₂₅.
5. The formulation according to claim 4, wherein the PTH is PTH₁₋₃₄.
6. The formulation according to claim 3, wherein the second domain comprises a transglutaminase substrate domain.
7. The formulation according to claim 6, wherein the transglutaminase domain comprises a Factor XIIIa substrate domain.
8. The formulation according to claim 3, wherein PTH fusion peptide further comprises a degradation site between the first and the second domains.
9. The formulation according to any of claims 1 to 8, wherein the granular material is a mixture of tricalcium phosphate and hydroxyapatite.
10. A kit comprising the formulation according to any of claims 1 to 9.
11. The kit according to claim 10, wherein the fibrinogen precursor component and thrombin precursor component are separated from each other.
12. The kit according to claim 10 or 11, wherein the PTH fusion peptide further comprises a degradation site between the first and the second domains, said degradation site being an enzymatic or hydrolytic degradation site.
13. A supplemented matrix comprising
   (i) PTH or a PTH fusion peptide;
   (ii) a granular material comprising a calcium mineral and
   (iii) fibrin;
   wherein said PTH or PTH fusion peptide is present in a concentration range of between 0.01 to 2 mg/mL fibrin matrix, with the proviso that a concentration of 0.4mg/mL fibrin matrix is not included.
14. The supplemented matrix according to claim 13, wherein the PTH is selected from the group consisting of PTH₁₋₈₄, PTH₁₋₃₈, PTH₁₋₃₄, PTH₁₋₃₁ and PTH₁₋₂₅.
15. The supplemented matrix according to claim 14, wherein the PTH is PTH₁₋₃₄.
16. The supplemented matrix according to any of claims 13 to 15, wherein the supplemented matrix is formed from a composition capable of forming a fibrin matrix and a fusion peptide, comprising the PTH in a first domain, and a covalently crosslinkable substrate domain in a second domain.
17. The supplemented matrix according to claim 16, wherein the second domain comprises a Factor XIIIa substrate domain.
18. The supplemented matrix according to any of claims 13 to 17, wherein the PTH fusion peptide further comprises a degradation site between the first and the second domains
19. The supplemented matrix according to claim 18, wherein the degradation site is an enzymatic degradation site.
20. The supplemented matrix according to any of claims 13 to 19, wherein the granular material is a mixture of tricalcium phosphate and hydroxyapatite.
21. Use of a supplemented matrix according to any one of claims 13 to 20 for the manufacture of a medicament to be locally administered for the repair of bone fractures.
22. Use of a formulation according to any one of claims 1 to 9 for the manufacture of a medicament to be locally administered for the repair bone fractures.
23. Use according to claim 21 or 22, wherein the bone fracture is a fracture of the distal radius or of the tibia.

## Claims

1. A supplemented matrix comprising
(i) PTH or a PTH fusion peptide; and
(ii) fibrin;
for use in the repair of bone fractures, in particular for use in repair of bone fractures comprises multiple bone fragments.

2. The supplemented matrix according to claim 1, wherein the use in the repair of bone fractures comprises the administration of a supplemented matrix locally to the site of the bone fracture.

3. The supplemented matrix according to any one of claims 1 or 2, wherein the bone fracture is at a bony site selected from the group consisting of the distal radius, the tibia and the hip.

4. The supplemented matrix according to any one of claims 1 to 3, wherein the use in the repair of bone fractures comprises implanting the matrix at the site of the bone fracture.

5. The supplemented matrix according to any one of claims 1 to 4, further comprising a granular material comprising a calcium mineral preferably chosen from the group consisting of hydroxyapatite, calcium phosphate and calcium sulphate and combinations thereof; in particular a mixture of tricalcium phosphate and hydroxyapatite.

6. A supplemented composition for use in treating bone fractures, in particular a matrix according to any one of claims 1 to 5, wherein the use in the repair of bone fractures comprises administration of the composition at the site of the bone fracture, in particular by injecting, wherein the composition comprises
(i) a peptide selected from the group consisting of PTH and a PTH fusion peptide; and
(ii) a composition capable of forming a fibrin matrix under physiological conditions comprising a first precursor solution comprising fibrinogen and a second precursor solution comprising thrombin.

7. The supplemented matrix according to claim 6, wherein the formulation further comprises a granular material comprising a calcium mineral preferably chosen from the group consisting of hydroxyapatite, calcium phosphate and calcium sulphate and combinations thereof; in particular a mixture of tricalcium phosphate and hydroxyapatite.

8. The supplemented matrix according to any one of claims 6 or 7, wherein the first precursor solution or the second precursor solution further comprises a calcium ion source.

9. The supplemented matrix according to any one of claims 1 to 8, wherein the PTH fusion peptide comprises at least two domains wherein the first domain comprises PTH and the second domain comprises a crosslinkable substrate domain.

10. The supplemented matrix of any one of claims 1 to 9, wherein the PTH is selected from the group consisting of PTH₁₋₈₄, PTH₁₋₃₈, PTH₁₋₃₄, PTH₁₋₃₁ and PTH₁₋₂₅.

11. The supplemented matrix of claim 9, wherein the second domain comprises a transglutaminase substrate domain, preferably a Factor XIIIa substrate domain.

12. The supplemented matrix of any one of claims 9 to 11, wherein the PTH fusion peptide further comprises a degradation site between the first and the second domains.

13. The supplemented matrix of any one of claims 1 to 12, wherein the site of the bone fracture is the tibia.

14. The supplemented matrix of any one of claims 1 to 13, wherein the PTH or PTH fusion peptide is present in a concentration range of between 0.01 to 2 mg/mL fibrin matrix, with the proviso that a concentration of 0.4 mg/mL fibrin matrix is not included.

15. The supplemented matrix of any one of claims 1 to 14, wherein the use in the repair of bone fractures comprises unifying the bone fragments and restoring the bone function.
